Europäisches Patentamt

European Patent Office

Office européen des brevets

(19)

(11) Veröffentlichungsnummer: **0 217 056**
**B1**

(12) **EUROPÄISCHE PATENTSCHRIFT**

(45) Veröffentlichungstag der Patentschrift:
31.01.90

(51) Int. Cl.⁴: **A 61 M 15/00**

(21) Anmeldenummer: **86110531.0**

(22) Anmeldetag: **30.07.86**

(54) **Dampfinhaliergerät.**

(30) Priorität: **31.07.85 DE 3527444**

(43) Veröffentlichungstag der Anmeldung:
**08.04.87 Patentblatt 87/15**

(45) Bekanntmachung des Hinweises auf die Patenterteilung:
**31.01.90 Patentblatt 90/5**

(84) Benannte Vertragsstaaten:
**AT BE CH DE FR GB IT LI LU NL SE**

(56) Entgegenhaltungen:
**EP-A- 0 098 537**
**AT-B- 348 661**
**DE-C- 639 470**
**GB-A- 23 904**
**US-A- 1 914 094**
**US-A- 3 809 080**

(73) Patentinhaber: **PLANTORGAN WERK Heinrich G.E. Christensen KG, Hornbusch 1, D-2903 Bad Zwischenahn (DE)**

(72) Erfinder: **Fink, Ernst, Prof. Dr. med. Dr. rer. nat. habil., Stellhorner Strasse 1, D-2910 Westerstede-Giesselhorst (DE)**

(74) Vertreter: **Kinzebach, Werner, Dr., Patentanwälte Reitstötter, Kinzebach und Partner Sternwartstrasse 4 Postfach 86 06 49, D-8000 München 86 (DE)**

ACTORUM AG

**Beschreibung**

Die Erfindung betrifft ein Dampfinhaliergerät nach dem Oberbegriff des Hauptanspruches.

Aus der Japanischen Gebrauchsmusteranmeldung JP-U-156640/84 ist ein Dampfinhaliergerät bekannt, das einen Behälter mit kegelförmigem Deckel und eine auf diesen aufgesetzte Atemmaske aufweist. Der Deckel weist eine Einsecköffnung auf, über die eine Belüftungsvorrichtung in Form eines Rohres mit Ausströmöffnung derart eingesetzt werden kann, daß die Ausströmöffnungen in der im Behälter enthaltenen Heilflüssigkeit liegen. Durch Gaszufuhr wird dann eine verbesserte Verdampfung der Heilflüssigkeit erzielt.

Um eine erhöhte Verdampfungsrate zu erzielen, wird in der DE-A-3 224 849 vorgeschlagen, in dem Behälter eine Zerstäubungsvorrichtung anzuordnen, mittels derer über eine gesonderte Druckluftzufuhr Heilflüssigkeit, die nicht notwendigerweise mit der Heilflüssigkeit im Behälter identisch ist, zerstäubt werden kann. Füllt man nun den Behälter mit heißer Flüssigkeit (Wasser mit oder ohne pharmazeutisch wirksamen Zusätzen) so erhält man zunächst angewärmte, stark befeuchtete Luft, die dann durch zusätzliche zerstäubte Heilflüssigkeit weiter befeuchtet wird. Während der Behandlung werden Mund und Nase an die Atemmaske fest angedrückt, so daß die heißen Dämpfe eingeatmet werden können. Diese bekannte Vorrichtung weist jedoch insbesondere den Nachteil auf, daß sie nur sehr schwer handhabbar ist. Dies ist ein wesentlicher Nachteil bei einem gattungsgemäßen Gerät, da Dampfinhaliergeräte insbesondere von kranken und gebrechlichen sehr oft von alten Menschen benutzt werden müssen. Insbesondere ist das bekannte Gerät deshalb so schwer zu handhaben, weil der Zerstäubereinsatz nur in äußerst komplizierter Weise in den Behälter eingesetzt werden kann und darüber hinaus die zusätzliche Heilflüssigkeit, die zerstäubt werden soll, nur sehr schwer einfüllbar ist.

Ein weiterer Nachteil liegt beim bekannten Gerät darin, daß die zerstäubte Heilflüssigkeit nur zu einem relativ geringen Teil in die Atemmaske gelangt, während ein großer Teil des Heilflüssigkeitsnebels zum Boden, d.h. in die (heiße) Flüssigkeit im Behälter fällt und sich nicht mit der vom Benutzer angesaugten Luft vermischt.

In der EP-A-98 537 wird ein Dampfinhaliergerät mit einem Behälter für zu verdampfende Heilflüssigkeit und einem kegelförmigen Deckel, der an seinem spitzen Ende eine Mund/oder Nase umschließende Atemmaske aufweist, vorgeschlagen. Eine Zerstäubervorrichtung ist innerhalb des kegelförmigen Deckels angeordnet. Der eigentliche Zerstäubereinsatz wird in die Zerstäubervorrichtung eingelegt und die Atemmaske darübergestülpt.

Ausgehend vom oben genannten Stand der Technik ist es somit Aufgabe der vorliegenden Erfindung ein Dampfinhaliergerät derart weiterzubilden, daß es sich leichter handhaben läßt und gleichzeitig der Wirkstoff sich besser in der Inhalationsluft verteilen läßt.

Diese Aufgabe wird bei einem Dampfinhaliergerät der eingangs genannten Art dadurch gelöst, daß die Zerstäubungsvorrichtung zwischen dem Deckel und der Atemmaske angeordnet ist und die Zerstäubungsvorrichtung einen Außenmantel mit zylindrischen Endabschnitten aufweist, die mit korrespondierend geformten Endabschnitten am zulaufenden Ende des Deckels bzw. an der Atemmaske in dichten Reibschluß bringbar sind.

Weiter ist die Zerstäubungsvorrichtung mit einem Düsenkegel versehen, der nach oben spitz zuläuft. Auf seiner Mantelfläche weist der Düsenkegel über die gesamte Höhe mindestens eine Nut auf, an seinem Oberende ist eine Düsenbohrung vorgesehen, die in gasdichter Verbindung mit einem Anschlußstutzen für Druckluft steht. Die Druckluft kann beispielsweise über einen Blasebalg erzeugt werden. Weiterhin ist ein oben offener Wirkstoffbehälter vorgesehen, aus dessen Bodenfläche der Kegel hervorsteht. Zwischen der Mantelfläche des Wirkstoffbehälters und dem Außenmantel sind Durchlässe angebracht, so daß angewärmte Luft aus dem Behälter in die Atemmaske eingesaugt werden kann. Weiterhin ist ein komplementär zum Kegel geformtes Abdeckteil auf den Kegel abnehmbar aufgesetzt, das mit dem Oberende des Kegels, jedoch nicht mit dessen Unterende im wesentlichen bündig abschließt. Durch dieses Teil ist gewährleistet, daß die Nuten Leitungen zwischen dem bodennahen Bereich des Wirkstoffbehälters und dem Oberende des Kegels bilden. Durch diese Maßnahmen wird erreicht, daß zum einen die Zerstäubungsvorrichtung leicht zugänglich ist und mit der zusätzlichen Heilflüssigkeit gefüllt werden kann, wenn der Behälter bereits befüllt ist. Zum anderen führt diese Anordnung dazu, daß der gesamte, aus dem Behälter angesaugte (temperierte) Luftstrom an der Zerstäubungsvorrichtung vorbeiströmt und die Flüssigkeitsteilchen mitnimmt, so daß diese ohne Verlust in die Atemwege des Benutzers gelangen. Ferner ist gewährleistet, daß die Zerstäubungsvorrichtung vollständig vom Deckel bzw. der Atemmaske getrennt werden kann, um sie zum einen zu befüllen, zum anderen nach der Benutzung reinigen zu können. Diese Reinigung ist bei einem Dampfinhaliergerät bekanntermaßen notwendig und muß ohne größeren Aufwand durchführbar sein.

Sobald nun Luft aus der Düse ausgeblasen wird, ergibt sich am Oberende der aus den Nuten gebildeten Leitungen ein Unterdruck, so daß die Wirkstoffzubereitung aus dem Wirkstoffbehälter angesaugt und mit der Luft vernebelt wird. Dadurch, daß die Nuten nur durch das Abdeckteil zur Bildung von Leitungen verschlossen sind, ist gewährleistet, daß man die gesamte Zerstäubungsvorrichtung nach dem Gebrauch gründlich reinigen kann. Der Zusammenbau ist denkbar einfach, da durch die komplementäre Formung von Kegel und Abdeckteil das Abdeckteil ohne zusätzliche Justiermaßnahmen ganz einfach auf den Kegel aufgesetzt werden kann, wenn die Zerstäubungsvorrichtung zusammengebaut werden soll.

Vorzugsweise ist der Düsenkegel mit dem Wirkstoffbehälter und einem ersten Außenmantelteil zur Bildung eines ersten Düsenträgerteils einstückig geformt, während ein zweites einstückig geformtes Düsenträgerteil vorgesehen ist, das an seinem Oberende einen Mantelring aufweist, der im wesentlichen bündig mit dem Unterende des ersten Außenmantelteils verklebt ist. Der Mantelring ist über einen Boden mit mindestens einer Ausnehmung mit einem zweiten Außenmantelteil verbunden, wobei im Boden Leitungsmittel eingeformt sind, die in den Anschlußstutzen übergehen und in dichter Strömungsverbindung der Düsenöffnung stehen. Durch diese kostengünstige Ausbildung der Zerstäubungsvorrichtung ist gewährleistet, daß nur die Teile, die tatsächlich gereinigt werden müssen, leicht zugänglich bzw. auseinandernehmbar sind, während z.B. die Druckluftleitung, die ja nicht verschmutzt, geschlossen bleibt. Auf diese Weise kann beim Zerlegen und Wiederzusammenbau des Dampfinhaliergerätes kein Fehler vorkommen.

Vorzugsweise weist das Abdeckteil an seinem Unterende einen ringförmigen Boden mit mindestens einer Öffnung auf, der an seinem äußeren Rand einen das Abdeckteil umgebenden und dieses überragenden Abdeckmantel trägt. Durch diese Anordnung ist gewährleistet, daß Flüssigkeitströpfchen mit zu großem Volumen nicht in den Luftstrom gelangen, sondern an den Abdeckmantel prallen, an diesem herablaufen und durch die Öffnung im Boden wieder in den Wirkstoffbehälter zurückfließen, um erneut zerstäubt werden zu können. Somit kommt aus dieser Zerstäubungsvorrichtung lediglich der feine Anteil (Aerosol) der zerstäubten Flüssigkeit, während die groben Flüssigkeitströpfchen wieder der zu zerstäubenden Flüssigkeit zugeführt werden können.

Über dem Abdeckmantel ist vorteilhafterweise ein Prallelement über Verbindungsmittel fest angeordnet. Die Anordnung ist derart getroffen, daß die Unterfläche des Prallelementes in im wesentlichen geringem Abstand über dem Oberende des Düsenkegels hängt. Durch diese Anordnung ergibt sich eine radiale Ausströmung des zerstäubten Nebels, was die oben erwähnte Auftrennung in Aerosolteilchen und grössere Tröpfchen bzw. deren Wiederverwendung begünstigt.

Weitere bevorzugte Ausführungsformen der Erfindung ergeben sich aus den nachfolgenden Ausführungsbeispielen, die anhand von Abbildungen näher erläutert sind.

Hierbei zeigt:

Figur 1 ein Dampfinhaliergerät, wie es bereits bekannt ist,

Figur 2 eine bevorzugte Ausführungsform des erfindungsgemäßen Dampfinhaliergerätes in einem Längsschnitt,

Figur 3 eine bervorzugte Ausführungsform der Zerstäubungsvorrichtung im Längsschnitt,

Figur 4 die Zerstäubungsvorrichtung nach Fig. 3 in einer Explosionsdarstellung,

Figur 5 eine Ansicht auf den Wirkstoffbehälter aus Fig. 4 entlang der Linie V-V aus Fig. 4

Figur 6 eine Ansicht entlang der Linie VI-VI aus Fig. 4, und

Figur 7 eine Ansicht entlang der Linie VII-VII aus Fig. 4 und

Figur 8 eine weitere Ausführungsform der Halterung eines Prallelementes.

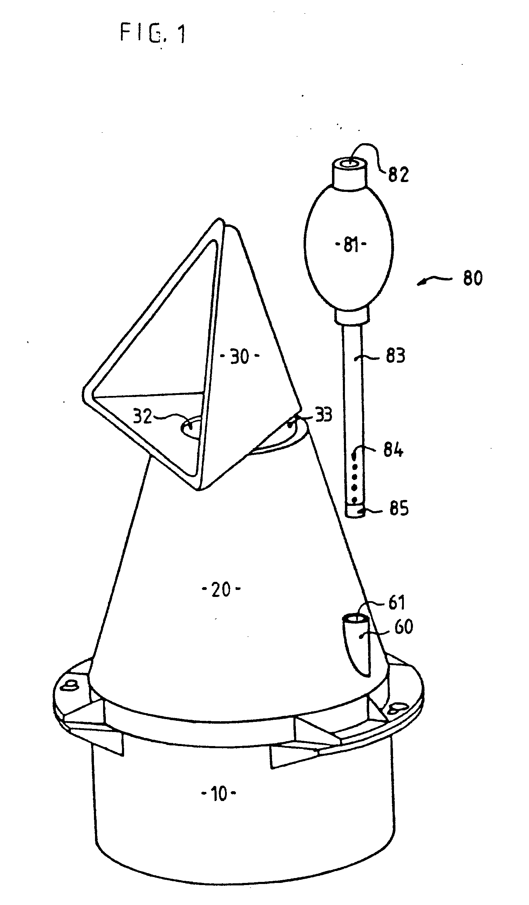

Fig. 1 zeigt ein Dampfinhaliergerät, wie es aus der japanischen Gebrauchsmusteranmeldung Nr. 156640/84 bekannt ist. Das Inhaliergerät weist hierbei einen Behälter 10 auf, auf dem ein kegelförmiger Deckel 20 sitzt. Der Behälter 10 und der Deckel 20 sind im Bereich von Griffplatten am Behälter und am Deckel über einen Bajonettverschluß miteinander lösbar verbunden. Der Deckel 20 weist Rohrstutzen 60 auf, deren Öffnung 61 mit dem Behälterinneren kommuniziert und im wesentlichen vertikal ausgerichtet ist. Auf dem Deckel 20 ist gegenüber dem Behälter 10 eine Atemmaske 30 aufgesetzt und zwar über ein Zwischenstück 33, das als Kugelgelenk ausgebildet ist und somit der Maske 30 eine gewisse Beweglichkeit erlaubt. Im Boden der Maske 30 ist eine Öffnung 32 ausgebildet, über welche das Innere der Maske 30 mit dem Inneren des Behälters 10 mit Deckel 20 kommuniziert. In die Öffnung 61 des Stutzens 60 im Deckel 20 kann eine Belüftungsvorrichtung 80 eingesetzt werden, die aus einem Rohr 83 besteht, das an seinem Ende mit einem Stopfen 85 verschlossen ist. In der Nähe des Stopfens 85 sind mehrere Luftaustrittsbohrungen 84 angebracht. Gegenüber dem Stopfen 85 ist ein Balg 81 auf das Rohr 83 aufgesetzt, der an seinem dem Rohr 83 gegenüberliegenden Ende ein Ventil 82 aufweist. Ist die Belüftungsvorrichtung 80 in den mit Flüssigkeit gefüllten Behälter 10 eingesetzt, so kann man über den Balg 81 Luft in die Flüssigkeit blasen, um die Verdampfungsrate beim Inhalieren zu vergrößern.

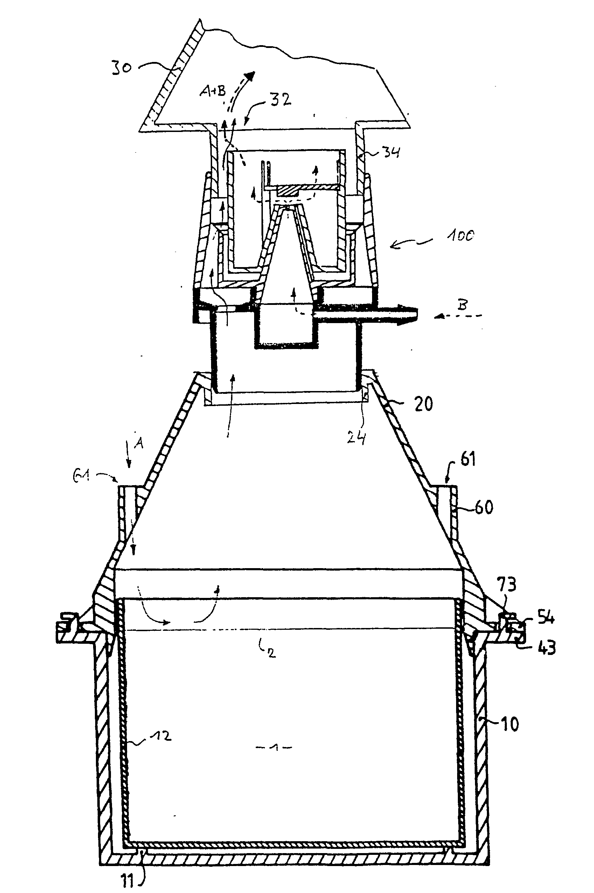

Im folgenden wird die Erfindung anhand der Fig. 2 näher beschrieben.

Der Behälter 10 mit Deckel 20 kann in der bekannten Weise ausgeführt sein, auch die Atemmaske 30 kann im wesentlichen gleichgestaltet sein. Wie aus Fig. 2 hervorgeht, befindet sich im Inneren des Behälters 10 ein topfförmiger Einsatz 12, der über Füßchen 11 an der Innenseite des Behälters 10 in Abstand zu diesem gehalten wird. In diesen Inneneinsatz 12 füllt der Benutzer heißes Wasser, dem gegebenenfalls eine pharmazeutisch wirksame Substanz beigemengt ist. Durch den Abstand zwischen dem Inneneinsatz 12 und dem Behälter 10 ist ein geringer Wärmeverlust gewährleistet.

Der Behälter 10 weist nach außen stehende Tragegriffe 43 auf, denen korrespondierend geformte radial hervorstehende Ansätze 54 am Deckel 20 entsprechen. Der Deckel 20 ist auf den Behälter 10 über einen Bajonettverschluß mit Haken 73 befestigt.

An seinem Oberende weist der Deckel 20 einen nach Innen ragenden Rohrstutzen 24 auf. In diesen Rohrstutzen 24 wird bei dieser bevorzugten Ausführungsform der Erfindung die Zerstäubungsvorrichtung 100 mit ihrem Unterende, das ebenfalls rohrförmig gestaltet ist, eingesetzt. In das Oberende der Zerstäubungsvorrichtung 100 wird der stutzenförmige Ansatz 34 der Atemmaske 30 gesteckt. Somit besteht eine im wesentlichen gasdichte Verbindung zwischen dem Inneren der Atemmaske 30 und dem Deckel 20 bzw. Behälter 10/12. Diese Verbindung führt ausschließlich durch die Zerstäubungsvorrichtung 100.

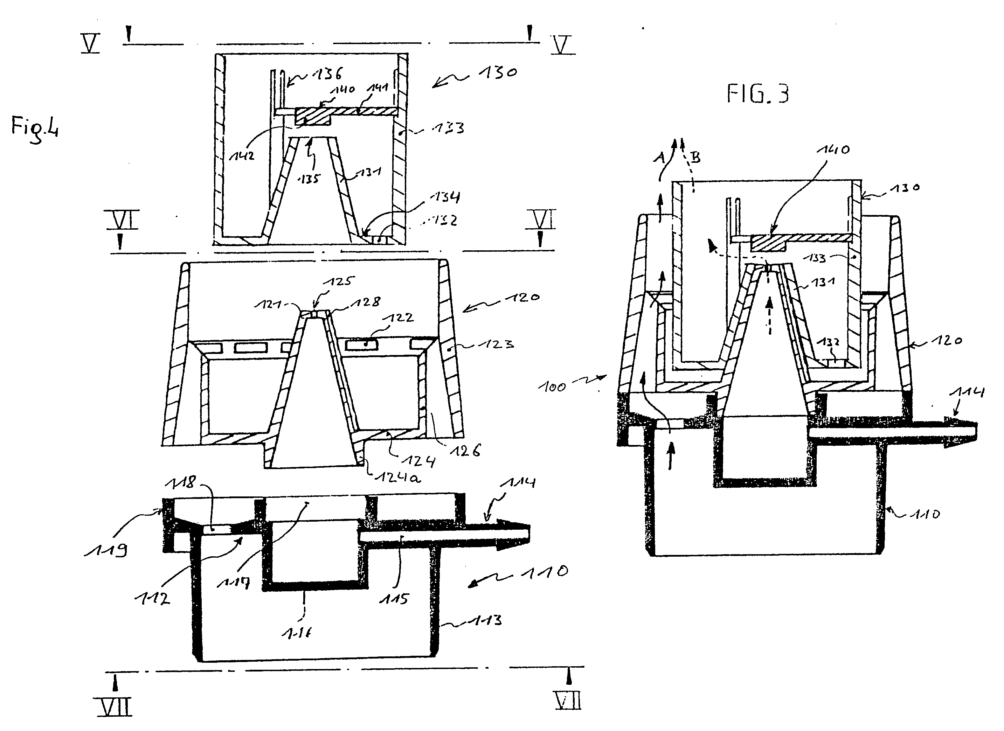

Im folgenden wird die Zerstäubungsvorrichtung 100 anhand der Fig. 3 bis 7 näher erläutert.

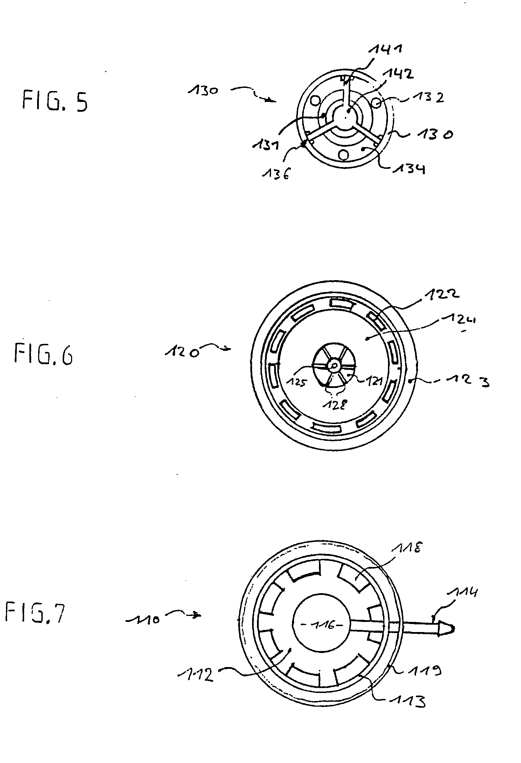

Wie den Fig. 3 und 4 zu entnehmen ist, besteht die Zerstäubungsvorrichtung 100 im wesentlichen aus drei Formteilen, die vorteilhafterweise aus Kunststoff spritzgeformt sind. Von diesen drei Formteilen sind wiederum zwei Formteile durch Reibschluß fest miteinander verbunden.

Diese beiden miteinander verbundenen Teile sind das erste Düsenträgerteil 120 und das zweite Düsenträgerteil 110. Das erste Düsenträgerteil 120 weist innen einen Düsenkegel 121 auf, der an seiner Außenfläche mit Nuten 128 versehen ist. Die Nuten laufen hierbei geradlining über die Mantelfläche von unten nach oben, so daß oben die Nuten in axialer Richtung offen sind. Der Düsenkegel 121 geht an seiner Basisfläche in eine Bodenfläche 124 über, die sich in der Normalebene zur Symmetrieachse des Düsenkegels 121 als Ring erstreckt. Am Außenumfang geht die Bodenfläche 124 in eine Mantelfläche 126 über, die konzentrisch zur Symmetrieachse des Düsenkegels 121 den Kegel teilweise überdeckend angeordnet ist.

Rings um den Kegel 121 bzw. die Mantelfläche 125 ist ein Außenmantel bzw. ein erstes Mantelteil 123 (konzentrisch) angeordnet. Zwischen der Mantelfläche 126 und dem Außenmantel 123 ist eine Verbindungszone vorgesehen, die von Durchlässen 122 durchbrochen ist. Auf diese Weise ist eine Gasströmung zwischen der Mantelfläche 126 und dem Außenmantel 123 dieses Teiles möglich. Der Außenmantel 123 ist hierbei so lang, daß die Düsenbohrung 125 im Düsenkegel 121 tiefer liegt als der obere Rand des Außenmantels 123. Am unteren Rand schließt der Außenmantel 123 mit der Bodenfläche 124 bündig ab.

Der Düsenkegel 121 ist als Hohlkegel (mit oben liegender Düse 125) ausgestaltet. Von der Bodenfläche 124 ragt ein Stutzen 124a mit im wesentlichen zylindrischer Außenkontur hervor, dessen Innenkontur den kegelförmigen Innenraum des Düsenkegels 121 fortsetzt. Dieses erste Düsenträgerteil 120 wird auf das zweite Düsenträgerteil 110 aufgesteckt.

Das zweite Düsenträgerteil 110 weist einen Mantelring 119 auf, dessen Außendurchmesser und dessen Wandstärke denen des oben beschriebenen ersten Mantelteils 123 entsprechen, so daß die beiden Teile miteinander dicht verbunden werden können. Der Mantelring 119 ist über einen Boden 112 mit darin befindlichen Ausnehmungen 118 mit einem topfförmigen Einsatz verbunden, der aus einem Topf 116 besteht, der an seinem, dem ersten Düsenträgerteil 120 zugewandten Ende einen Ringstutzen 117 aufweist. Der Ringstutzen 117 weist einen Innendurchmesser auf, der dem Rohrstutzen 124a des ersten Düsenträgerteils 120 entspricht. Wird das erste Düsenträgerteil 110 auf das zweite Düsenträgerteil 120 aufgesetzt, so gelangt also der Rohrstutzen 124a in den Ringstutzen 117 und dichtet den Innenraum des Düsenkegels 121 mit dem Innenraum des Topfes 116 ab. Vom Topf 116 führt in radialer Richtung eine Leitung 115 nach außen, die in einen Anschlußstutzen 114 übergeht. An den Anschlußstutzen 114 kann z.B. über eine Schlauchleitung ein Blasebalg angekoppelt werden. Im zusammengebauten Zustand (Fig. 3) ist somit eine Luftströmung, wie mit den Pfeilen A angedeutet, durch die Zerstäubungsvorrichtung 110 möglich.

Auf den Düsenkegel 121 ist ein Düsenteil 130 aufgesetzt, das als wichtigstes Teil das Abdeckteil 131 aufweist. Das Abdeckteil 131 ist mit einer Innenausnehmung versehen, die komplementär zum Düsenkegel 121 geformt ist. Hierbei ist die Oberöffnung 135 des Abdeckteils 131 mit demselben Durchmesser ausgebildet, wie ihn der Düsenkegel 121 auf seiner Endfläche aufweist, in der sich die Düsenbohrung 125 befindet. Die Länge des Abdeckteils 131 ist hierbei so bemessen, daß die Nuten 128 bei aufgesetztem Abdeckteil 131 in der Nähe des Bodens 124 frei zugänglich sind, während das Abdeckteil 131 im wesentlichen zusammen mit dem Düsenkegel 121 eine Endfläche bildet (siehe Fig. 3).

Das Abdeckteil 131 geht an seinem Unterende in einen Boden 134 über, der in der Normalebene zur Symmetrieachse des Abdeckteils 131 angeordnet ist. Im Boden 134 befinden sich Öffnungen 132. Der Boden 134 ist ringförmig ausgebildet und geht an seinem Außenrand in einen Abdeckmantel 133 über, der sich konzentrisch zur Symmetrieachse des Abdeckteils 131 nach oben erstreckt. Die Länge des Abdeckmantels 133 ist hierbei so bemessen, daß er ein ganzes Stück über das (spitze) Ende des Abdeckteils 131 nach oben ragt. Der Außendurchmesser des Abdeckmantels 133 ist so bemessen, daß zwischen Abdeckmantel 133 und Mantelfläche 126 des ersten Düsenträgerteils 120 zumindest ein gewisses Spiel besteht.

Auf der Innenfläche des Abdeckmantels 133 sind Haltenuten 136 vorgesehen, die in wulstförmigen Erhebungen auf der Innenfläche des Abdeckmantels 133 angeordnet sind. Die Nuten 136 verlaufen hierbei im wesentlichen parallel zur Symmetrieachse des Düsenteils 130 und sind nach außen offen, während ihre dem Boden 134 zugewandten Enden in einem definierten Abstand zum Boden 134 angeordnet sind. Vorzugsweise sind drei derartige Nuten vorgesehen.

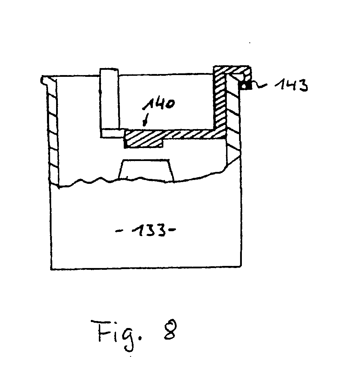

In den Nuten 136 sitzen die Außenenden von radial sich erstreckenden Streben 141, die ein im wesentlichen zylindrisches Prallelement 140 tragen. Das Prallelement 140 und die Tiefe der Nuten 136 sind hierbei so bemessen, daß die Unterfläche 142 des Prallelementes 140 knapp (0,5 bis 2,5 mm) über dem Ende des Abdeckteils 131 hängt. Das Prallelement bzw. die Streben 141 sind in den Nuten 136 fixiert, z.B. durch Kleben oder Schweißen. Somit bildet das Düsenteil 130 zusammen mit dem Prallelement im fertigen Zustand des Gerätes ein nur in dieser Verbindung handhabbares Teil. Bei einer weiteren bevorzugten Ausführungsform (Fig. 8) ist das Prallelement 140 über Haken 143 am Oberrand des Abdeckmantels 133 (formschlüssig) eingehängt.

Setzt man das Düsenteil 130 wie in Fig. 3 gezeigt auf den Düsenkegel 121, so ist die Zerstäubungsvorrichtung 100 automatisch richtig justiert, da der Sitz über zwei konische Flächen erfolgt.

Im folgenden wird die Wirkungsweise des erfindungsgemäßen Dampfinhaliergerätes gemäß der beschriebenen bevorzugten Ausführungsform näher erläutert. Zunächst trennt der Benutzer den Deckel 20 vom Behälter 10 und füllt den Innenbehälter 12 mit Heilflüssigkeit 1 derart auf, daß der Flüssigkeitsspiegel 2 etwas unter dem Oberrand des Innenbehälters 12 steht. Dann setzt der Benutzer den Deckel 20 auf den Behälter 10 auf und verriegelt ihn mit diesem. Daraufhin setzt der Benutzer die Zerstäubugsvorrichtung 100 fest in den Deckel 20 ein und füllt eine weitere pharmazeutisch wirksame Substanz in den Wirkstoffbehälter 124/126. Hierzu muß das Düsenteil 130 nicht extra abgenommen werden, da die Heilflüssigkeit auch durch die Öffnung 132 im Boden 134 des Düsenteils 130 in den Wirkstoffbehälter 124/126 gelangen kann. Danach (gegebenenfalls nachdem weiterhin das Düsenteil 130 aufgesetzt wurde) setzt der Benutzer die Atemmaske 30 mit ihrem Stutzen 34 in das Oberende der Zerstäubungsvorrichtung 100 ein. Danach bildet das Dampfinhaliergerät eine im wesentlichen fest verbundene Einheit.

Atmet nun der Benutzer durch die Atemmaske 30 ein, so strömt Luft durch die Öffnungen 61 im Deckel 20 in Richtung auf die Flüssigkeitsoberfläche 2. Dort wird die Luft von der Flüssigkeit 1 befeuchtet und angewärmt. Die so befeuchtete Luft gelangt durch die Öffnungen 118 im Boden 112 des zweiten Düsenträgerteils in den Zwischenraum zwischen Mantelfläche 126 und Außenmantel 123 des ersten Düsenträgerteils. Durch die Durchlässe 122 im ersten Düsenträgerteil 120 strömt dann die Luft zwischen dem Abdeckmantel 133 und dem Rohrstutzen 34 in die Atemmaske 30.

Führt nur der Benutzer dem Anschlußstutzen 114 Druckluft zu, so strömt diese Druckluft (strichpunktierte Pfeile B) durch die Düsenbohrung 125 aus. Durch die erhöhte Strömungsgeschwindigkeit der Luft in diesem Bereich entsteht ein Unterdruck, der über die von den Nuten 128 gebildeten Leitungen zum Boden 124 des Wirkstoffbehälters 124/126 geleitet wird. Durch diesen Unterdruck wird Heilflüssigkeit, die im Wirkstoffbehälter 124/126 angeordnet ist, angesaugt und in Richtung auf die Spitze des Düsenkegels 121 transportiert. Dort wird die angesaugte Heilflüssigkeit mit der aus der Düse 125 ausströmenden Luft verwirbelt und versprüht so in Tropfen kleinen Durchmessers. Durch das Prallelement 140 wird der so entstehende Nebel radial nach außen geleitet, in Richtung auf den Abdeckmantel 133. Die feinen Tröpfchen des entstandenen Wirkstoffnebels werden mit der durch den Sstutzen 114 zugeführten Druckluft aus dem Düsenteil 130 nach oben getragen. Größere Teilchen sinken ab bzw. rinnen am Abdeckmantel 133 nach unten, um sich dort wieder mit der Heilflüssigkeikt im Wirkstoffbehälter 124/126 zu vereinigen. Die feinen Tröpfchen jedoch werden mit der auf der Außenseite des Abdeckmantels 133 vorbeiströmenden angesaugten Luft gründlich verwirbelt, da diese Luft aufgrund des Strömungsweges eine relativ hohe Eigengeschwindigkeit, sowie eine starke Turbulenz aufweist. Durch die hier gezeigte Anordnung wird also eine besonders gute Verwirbelung der zerstäubten Flüssigkeit erzielt.

Nach Ende der Behandlung zerlegt der Benutzer die Vorrichtung, indem er ganz einfach die Atemmaske 30 von der Zerstäubungsvorrichtung 100 abnimmt, die Zerstäubungsvorrichtung 100 insgesamt vom Deckel 20 abnimmt und in ein Wasserbad zur Reinigung legt, worauf er den Deckel 20 entfernen und den Inneneinsatz 12 herausnehmen und entleeren und ebenfalls reinigen kann. Nach dem Reinigen und Trocknen kann dann wieder jederzeit mit nur geringem Aufwand eine neue Behandlung vorgenommen werden.

**Patentansprüche**

1. Dampfinhaliegerät mit einem Behälter (10) für zu verdampfende Flüssigkeit, einem kegelförmigen Deckel (20), einer Atemmaske (30) und einer Zerstäubungsvorrichtung (100), die für eine Wirkstoffzubereitung vorgesehen ist, dadurch gekennzeichnet, daß

a) die Zerstäubungsvorrichtung (100) zwischen dem Deckel (20) und der Atemmaske (30) angeordnet ist und einen Außenmantel (113, 119, 123) mit zylindrischen Endabschnitten aufweist, die mit korrespondierend geformten Endabschnitten (24, 34) am zulaufenden Ende des Deckels (20) bzw. an der Atemmaske (30) in dichten Reibschluß bringbar sind,

b) die Zerstäubungsvorrichtung (100) einen Düsenkegel (121) aufweist, der nach oben spitz zuläuft, auf seiner Mantelfläche über die Gesamthöhe midestens eine Nut (128) und an seinem Oberende eine Düsenbohrung (125), die in gasdichter Verbindung mit einem Anschlußstutzen (114) für Druckluft steht, aufweist,

c) die Zerstäubungsvorrichtung einen nach oben offenen Wirkstoffbehälter (124/126) aufweist, aus dessen Bodenfläche (124) der Kegel (121) hervorsteht und zwischen dessen Mantelfläche (126) und dem Außenmantel (123) Durchlässe (122) angebracht sind,

d) und daß weiterhin ein komplementär zum Kegel (121) geformtes Abdeckteil (131) auf den Kegel (121) abnehmbar aufgesetzt ist, das mit dem Oberende des Kegels (121), jedoch nicht mit dessen Unterende im wesentlichen bündig abschließt, so daß die Nuten (128) Leitungen zwischen dem bodennahen Bereich des Wirkstoffbehälters (124/126) und dem Oberende des Kegels (121) bilden.

2. Dampfinhaliergerät nach Anspruch 1, dadurch gekennzeichnet, daß der Düsenkegel (121) mit dem Wirkstoffbehälter (124/126) und einem ersten Außenmantelteil (123) zur Bildung eines ersten Düsenträgerteils (120) einstückig geformt ist, und daß ein zweites, einstückig geformtes Düsenträgerteil (110) vorgesehen ist, das an seinem Oberende einen Mantelring (119) aufweist, der im wesentlichen bündig mit dem Unterende des ersten Außenmantelteils (123) als Steckverbindung ausgebildet ist und der über einen Boden (112) mit mindestens einer Ausnehmung (118) mit einem zweiten Außenmantelteil (113) verbunden ist, wobei im Boden (112) Leitungsmittel (115 bis 117) eingeformt sind, die in den Anschlußstutzen (114) übergehen und in dichter Strömungsverbindung mit der Düsenöffnung (125) stehen.

3. Dampfinhaliergerät nach Anspruch 2, dadurch gekennzeichnet, daß das Abdeckteil (131) an seinem Unterende einen ringförmigen Boden (134) mit mindestens einer Öffnung (132) aufweist, der an seinem äußeren Rand einen das Abdeckteil umgebenden und dieses überragenden Abdeckmantel (133) trägt.

4. Dampfinhaliergerät nach Anspruch 3, dadurch gekennzeichnet, daß im Abdeckmantel (133) ein Prallelement (140) über Verbindungsmittel (141) derart angeordnet ist, daß seine Unterfläche in im wesentlichen geringem Abstand über dem Oberende des Düsenkegels (121) hängt.

## Claims

1. Vapour inhaling apparatus with a container (10) for the liquid which is to be vaporised, a conical cover (20), a breathing mask (30) and an atomising device (100) which is provided for an active substance preparation, characterised in that

a) the atomising device (100) is arranged between the cover (20) and the breathing mask (30) and has an outer casing (113, 119, 123) with cylindrical end portions which can be brought into close frictional contact with correspondingly shaped end portions (24, 34) at the converging end of the cover (20) or on the breathing mask (30),

b) the atomising device (100) has a nozzle cone (121) which converges to a point in the upward direction and on its outer surface over the entire height thereof it has at least one groove (128), and at its upper end a nozzle bore (125) which forms a gas-tight connection with a connector (114) for compressed air,

c) the atomising device has an upwardly open container (124, 126) for active substance, the cone (121) projecting from the base surface (124) thereof whilst between this outer surface (126) and the outer casing (123) are provided openings (122),

d) and furthermore a covering part (131) which is shaped to complement the cone (121) is removably placed on the cone (121) and forms a substantially flush joint with the upper end of the cone (121) but not with the lower end thereof, so that the grooves (128) form conduits between the region of the active subtance container (124/126) close to the base and the upper end of the cone (121).

2. Vapour inhaling apparatus according to claim 1, characterised in that the nozzle cone (121) is integrally formed with the active substance container (124/126) and a first outer casing part (123) to form a first nozzle carrier part (120), and a second integrally formed nozzle carrier part (110) is provided which has at its upper end an outer ring (119) which is formed subtantially flush with the lower end of the first outer casing part (123) as a push-in connection and which is connected to a second outer casing part (113) via a base (112) having at least one recess (118), whilst in the base (112) are formed conveying means (115 to 117) which merge into the connector (114) and are tightly connected for flow with the nozzle opening (125).

3. Vapour inhaling apparatus according to claim 2, characterised in that the covering part (131) has at its lower end an annular base (134) having at least one opening (132) which has at its outer edge a covering sleeve (133) which surrounds the covering part und projects over it.

4. Vapour inhaling apparatus according to claim 3, characterized in that an impact element (140) is arranged in the covering sleeve (133) with the aid of connecting means (141) so that its lower surface is suspended above the upper end of the nozzle cone (121) at a substantially small spacing therefrom.

## Revendications

1. Inhalateur de vapeur équipé d'un récipient (10) pour le liquide à vaporiser, d'un couvercle conique (20), d'un masque respiratoire (30) et d'un dispositif de pulvérisation (100), qui est prévu pour une préparation de l'agent actif, caractérisé en ce que

a) le dispositif de pulvérisation (100) est disposé entre le couvercle (20) et le masque respiratoire (30) et comporte une enveloppe extérieure (113, 119, 123) avec des tronçons terminaux cylindriques, pouvant être verrouillés par friction de façon étanche avec des tronçons terminaux (24, 34) façonnées de manière complémentaire sur l'extrémité d'entrée du couvercle (20) ou sur le masque respiratoire (30),

b) le dispositif de pulvérisation (100) comporte un cône de buse (121), à pointe tournée vers le haut, qui présente sur sa surface latérale sur toute la hauteur au moins une gorge (128), et à son extrémité supérieure un orifice de buse (125) en liaison étanche au gaz avec une tubulure de raccordement (114) pour de l'air comprimé,

c) le dispositif de pulvérisation comporte un récipient (124/126) d'agent actif ouvert vers le haut, de la surfac de fond (124) duquel fait saillie le cône (121) et entre la surface latérale (126) duquel et l'enveloppe extérieure (123) sont pratiqués des passages (122),

d) et en ce qu'en outre une pièce de recouvrement (131) façonnée de façon complémentaire au cône (121) est posée de façon amovible sur le cône (121), et se termine sensiblement de niveau avec l'extrémité supérieure du cône (121), mais pas avec l'extrémité inférieure de celui-ci, de sorte que les gorges (128) forment des canalisations entre la région proche du fond du récipient (124/126) d'agent actif et l'extrémité supérieure du cône (121).

2. Inhalateur de vapeur selon la revendication 1, caractérisé en ce que le cône de buse (121) est réalisé d'une seule pièce avec le récipient (124/126) d'agent actif et une première partie d'enveloppe extérieure (123) en vue de la formation d'un premier support de buse (120), et en ce qu'un second support de buse (110) réalisé d'une seule pièce est prévu, qui présente à son extrémité supérieure une collerette (119), qui est réalisée sensiblement de niveau avec l'extrémité inférieure de la première partie d'enveloppe extérieure (123) sous la forme d'une liaison enfichable et qui est reliée par l'intermédiaire d'un fond (112) avec au moins un évidement (118) à une seconde partie d'enveloppe extérieure (113), des conduits (115 à 117) étant formés dans le fond (112) qui communiquent avec la tubulure de raccordement (114) et sont en liaison d'écoulement étanche avec l'orifice de buse (125).

3. Inhalateur de vapeur selon la revendication 2, caractérisé en ce que la partie de recouvrement (131) présente à son extrémité inférieure un fond annulaire (134) avec au moins une ouverture (132), qui porte à son bord extérieur une paroi latérale (133) entourant la pièce de recovrement et faisant saillie de celle-ci.

4. Inhalateur de vapeur selon la revendication 3, caractérisé en ce que dans la paroi latérale de recouvrement (133) est disposé un élément de rebondissement (140) par l'intermédiaire de moyens de liaison (141), de sorte que sa surface inférieure soit suspendue à un écartement sensiblement faible au-dessus de l'extrémité supérieure du cône de buse (121).

FIG. 1

Fig.4

FIG.3

FIG. 5

130

141
142
132
131
130
136
134

FIG. 6

120

122
124
125
121
128
123

FIG. 7

110

118
114
116
112
119
113

143

140

- 133 -

Fig. 8